# EUROPEAN PATENT SPECIFICATION

(11) **EP 0 423 335 B1**
(45) Date of publication and mention of the grant of the patent: **15.03.1995**
(21) Application number: 89906460.4
(22) Date of filing: 02.06.1989
(51) Int. Cl.: A61M 1/02, A61B 5/14

(54) **BLOOD COLLECTION APPARATUS**
BLUTSAMMELVORRICHTUNG
APPAREIL DE PRELEVEMENT DE SANG

(30) Priority: 29.06.1988 JP 159404/88; 29.06.1988 JP 159405/88
(43) Date of publication of application: 24.04.1991
(73) Proprietor: TERUMO KABUSHIKI KAISHA, Tokyo 151 (JP)
(72) Inventor: INABA, Fumiaki, Fujinomiya-shi,Shizuoka 418 (JP); INOUE, Satoshi, Fujinomiya-shi,Shizuoka 418 (JP)
(74) Representative: Casalonga, Axel
(86) International application number: JP8900555
(87) International publication number: WO9000068

(56) References cited:
- EP-A- 0 044 707
- FR-A- 2 353 840
- FR-A- 2 606 639
- JP-A- 6 323 644
- JP-B- 513 153
- US-A- 4 667 153
- US-A- 4 672 974

## Description

This invention relates to a blood collecting apparatus for collecting blood into a blood container.

Heretofore, there was proposed a blood collecting apparatus as described in Japanese patent publication No. Sho 51-3153.

A blood collecting apparatus, in general, must include a sensor for measuring the amount of blood collected in a blood container.

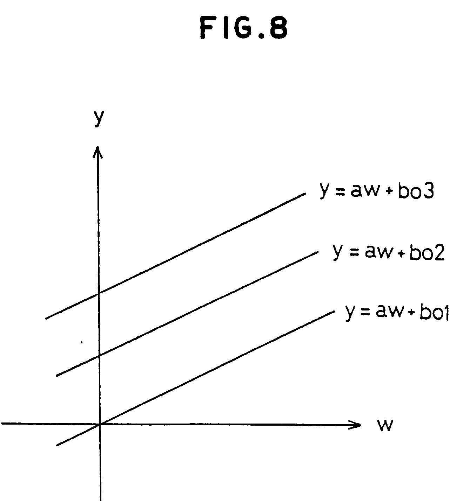

In a blood collecting apparatus for collecting blood in a blood container by negative pressure to be generated by a vacuum pump, the blood collecting apparatus must be provided with a pressure sensor for measuring the pressure in a blood collecting evacuated chamber where the blood container is to be set.
(I) The measuring sensor and pressure sensor can be handled in such a manner that the output of those sensors is in a certain relationship with the measured weight (amount of collected blood or pressure), which can he expressed as a constant function. Therefore, a blood collecting apparatus is provided with control means for calculating a desired measured weight (amount of collected blood or pressure) on the assumption that the output of the measuring sensor can be expressed as the above-mentioned specific function.
   Taking the sensor for measuring the amount of collected blood as also representing the other sensor, if it is handled in such a manner that the output y of the measuring sensor is in a certain relation to the measured weight w of collected blood, which can be expressed as the linear function y = aw + b (wherein a: gain [sensitivity] and b: offset value), the constants a and b are characteristics inherent to the individual sensors and thus different per each sensor from the beginning.
   Therefore, in a blood collecting apparatus using a sensor as stated above, it is required that the constants are properly changed for correction during the early stage of the use of such sensor and also in the transit stage of the use thereof, so that the amount of collected blood calculated by a control means based on the output of the sensor always coincides with a proper value.
   At that time, it can be considered that the correction of changing the constants a and b of the sensors is effected by providing a change circuit and that the output signals of the sensor are adjusted by the volume or the like. However, this idea has, for example, the following inconveniences. 10 a conversion circuit is required and 02 the volume, etc. must be adjusted in the manufacturing process and in the using process. FR-A-2 606 639 discloses a blood collecting apparatus, wherein the blood is set in blood collecting evacuated chamber and blood is collected into the blood container by negative pressure generated in the blood collecting chamber. A sensor including weight measuring means measures the amount of collected blood which is calculated by control means on the assumption that the output y of the measuring sensor is in a certain relationship with the measured weight w of collected blood, which can be expressed as a function y = f(w) comprising at least one constant value. This apparatus averages the inaccuracy of measurement by a formula taking into account the maximum and the minimum measuring values.
   An object of the present invention is to correct the characteristic of a sensor for measuring the amount of collected blood with ease and to obtain accuracy in the measurement of the amount of collected blood as well as to correct the characteristic of a pressure sensor and to obtain accuracy in the measurement of pressure generated in a blood collecting evacuated chamber.
(II) Similarly, taking the sensor for measuring the amount of collected blood as representing the other sensor, if it is handled in such a manner that the output y of the measuring sensor is in a certain relationship with the measured weight w of collected blood, which can be expressed as the linear function y = aw + b (wherein a: gain [sensitivity] and b: offset value), the offset value b sometimes drifts with the passage of time because of heat and change in stress as general characteristics of sensors (see Fig. 8).

Accordingly, when the offset value b01 of the linear function initialized in the control means should drift in such a way as b02, b03...during use, error values (b02-b01 ), (b03-b01 )...would occur unless they are corrected.

Further, the change caused by drift of the offset value of the output of a sensor for measuring the amount of collected blood should be automatically corrected to obtain accuracy in the measurement of the amount of collected blood.

As claimed the blood collecting apparatus according to the invention comprises a blood container which is set in blood collecting evacuated chamber and blood is collected into the blood container by negative pressure generated in the blood collecting evacuated chamber. The apparatus further is provided with a correcting function of a sensor including weight measuring means provided with a sensor for measuring the amount of collected blood when blood is collected in the blood container. Further, control means controls calculating the amount of collected blood on the assumption that the output y of the measuring sensor is in a certain relationship with the measured weight w of collected blood, which can be expressed as a function y = f(w) comprising at least one constant value. According to the invention the control means is provided with a memory for storing specific constant values of the sensor and in addition is provided with a correction mode, which may be adopted before starting or after completion of blood collection. In the correction mode n + 1 pieces of known amounts wi of collected blood are measured by the sensor and the output yi of the sensor is taken by the control means for correcting the constant value of the function y = f(w) and the corrected constant value is rewritten in the memory, thus correcting the characteristic of the sensor. As well, the control means is provided for calculating the weight of the collected blood using the corrected constant values. A pressure measuring means is also provided with a pressure sensor for detecting the pressure in the blood collecting evacuated chamber, the control means calculating pressure on the assumption that the output z of the pressure sensor is in a certain relationship with the measured pressure p, which can be expressed as a function z = f(p), comprising at least one constant value, the memory of the control means storing the corresponding specific constants of the pressure sensor. In the correction mode of the control means n + 1 pieces of known pressure amounts pi are measured by the pressure sensor and the output zi is taken by the control means for correcting the constant value and the corrected constant value is rewritten in the memory, thus correcting the characteristic of the pressure sensor.

According to the present invention the control means calculates the amount of collected blood from the output y of the measuring sensor based on the function y = f(w) established between the output y of the measuring sensor and the measured weight w (capacity or weight) of collected blood. At this time, the control means takes the output yi of the measuring sensor in n + 1 pieces of respective known amounts wi of collected blood up and corrects the constant constituting y = f(w) on the basis of such data taken up. Accordingly, it can correct the characteristic of the sensor for measuring the amount of collected blood with ease and obtain the accuracy in measurement of the amount of collected blood without requiring the conversion circuit and volume.

In addition to the calculation of the weight w of collected blood, the control means calculates the pressure p in the blood collecting evacuated chamber from the output z of the pressure sensor based on the function z = f(p) established between the output z of the pressure sensor and the measured pressure p. At that time, the control means takes the output zi of the pressure sensor in n + pieces of respective known pressure pi up and corrects the constant constituting z = f(p) on the basis of such data taken up. Accordingly, the control means can also correct the characteristic of the pressure sensor with ease and obtain the accuracy in measurement of pressure generated within the blood collecting evacuated chamber without requiring the conversion circuit and volume.

An advantageous embodiment of the blood collecting apparatus according to the invention, which is provided with an automatic correction function of the sensor has the output y0 of the measuring sensor automatically taken up every predetermined cycle in the state where the measured weight w of collected blood is zero and the off-set value of the function y = f(w) is corrected on the basis of y0.

Preferably, the measuring sensor is a weight sensor for measuring the weight of collected blood. the blood collecting apparatus further including pressure measuring means provided with a pressure sensor for detecting the pressure in the blood collecting evacuated chamber, the control means calculating the pressure on the assumption that the output z of the pressure sensor is in a certain relationship with the measured pressure p, which can be expressed by the function z = f(p), and automatically taking up the output z0 of the pressure sensor every predetermined cycle in the state where the measured pressure p is zero and correcting the offset value of the function z = f(p) on the basis of this output value z0.

According to the two latter embodiments of the present invention the control means calculates the weight w of collected blood from the output y of the pressure sensor based on the function y = f-(w) established between the output y of the measuring sensor and the measured weight w of collected blood (capacity or weight). At that time, the control means automatically takes up an output y0 of the measuring sensor every predetermined cycle in the state where the measured weight w of collected blood is zero and corrects the offset value of the function y = f(w) on the basis of this output value y0. That is, the control means can normally and automatically correct the changes caused by drift of the offset value of the output of the sensor for measuring the amount of collected blood and it can also obtain the accuracy in measurement of the amount or weight of collected blood.

In the following the invention is further elucidated by the description of preferred embodiments in conjunction with the drawings. In the drawings:
Fig. 1 is a front view showing a blood collecting apparatus in accordance with an embodiment of the present invention;
Fig. 2 is a partially cut-away side view showing essential parts of the apparatus shown in Fig. 1;
Fig. 3 is a plan view of the apparatus shown in Fig. 1;
Fig. 4 is a partially cut-away plan view showing essential parts of the apparatus shown in Fig. 1;
Fig. 5 is a view of a vacuum circuit;
Fig. 6 is a control block diagram;

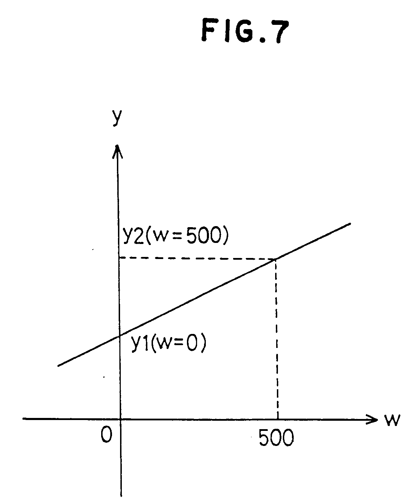

Fig. 7 is a diagram showing a method for determining the characteristic of a sensor; and
Fig. 8 is a diagram showing a drifted state when the sensor is offset.

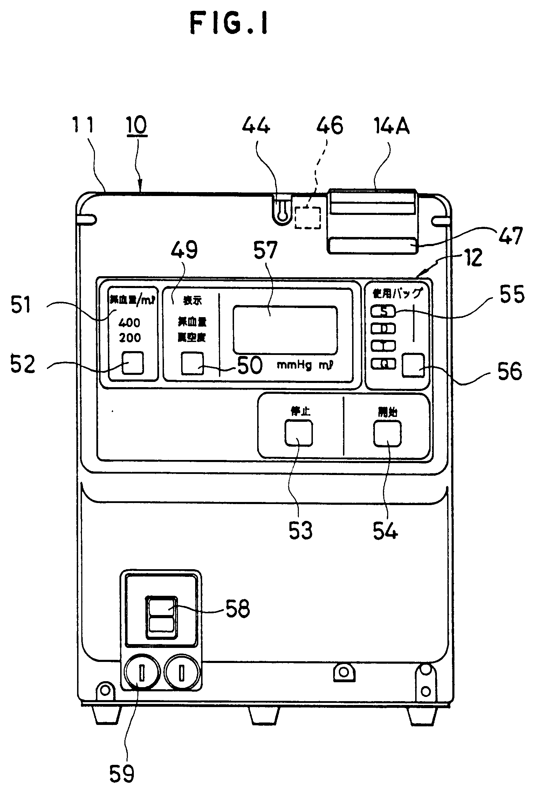

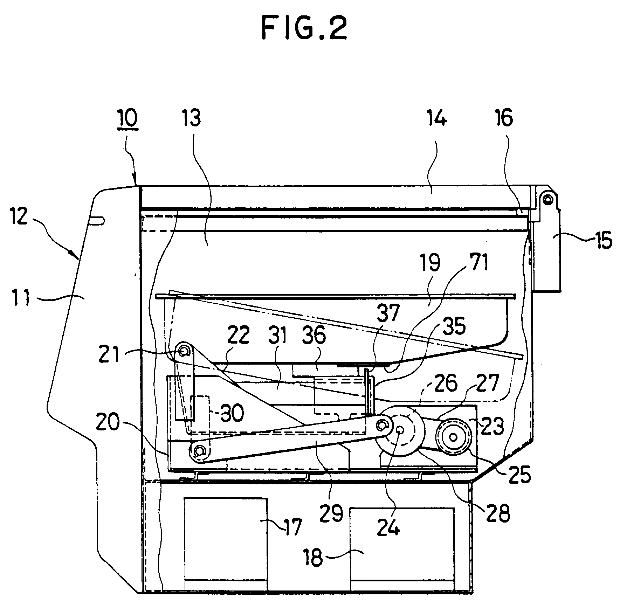

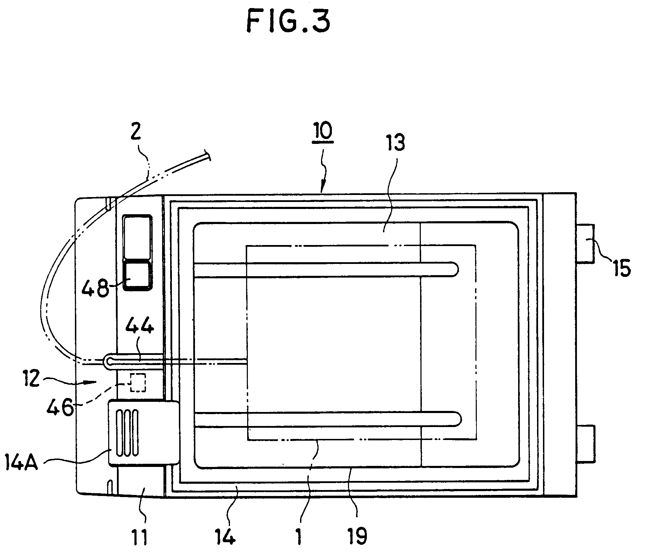

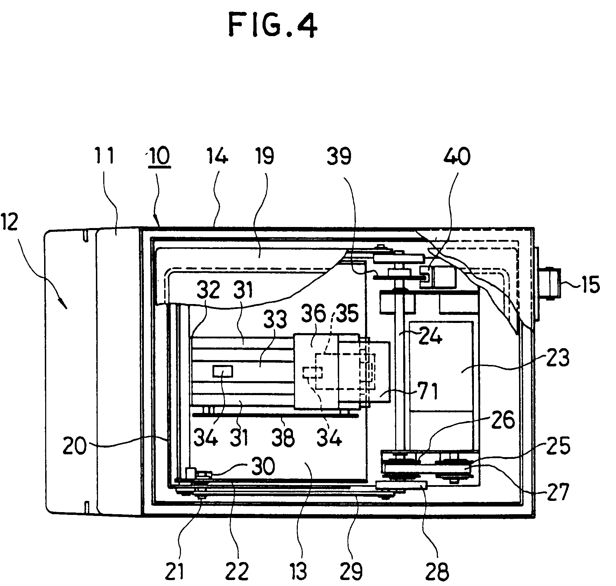

As shown in Figs. 1 to 4, a blood collecting apparatus 10 has a display panel 12 on the front surface of a housing 11, and a blood collecting evacuated chamber 13 formed inside the housing 11. Reference numeral 14 denotes a cover for opening and closing the blood collecting evacuated chamber 13; 15, a hinge for the cover 14; and 16, a sealing rubber for sealing the blood collecting evacuated chamber 13. Reference numeral 14A denotes a handle of the cover 14. The blood collecting apparatus 10 also has a vacuum pump 17 and a control device 18 accommodated within the housing 11 at lower positions thereof.

The blood collecting evacuated chamber 13 of the blood collecting apparatus 10 communicates with a suction port 17A of the vacuum pump 17 so as to enable pressure reduction, and the chamber is provided with a bag supporting plate 19 supporting a blood bag (blood container) 1 formed of polyvinyl chloride or the like. The blood collecting apparatus 10 collects blood while the blood collecting evacuated chamber 13 is in its pressure reduced condition, and while a predetermined negative pressure is applied to the blood bag 1 supported by the bag supporting plate 19. During this time, the blood collecting apparatus 10 operates to swing the bag supporting plate 19 intermittently for periods in a predetermined cycle so as to agitate blood together with an anticoagulant, such as heparin, already charged in the blood bag 1, and the apparatus also operates to measure the amount of collected blood by measuring the weight of the blood bag 1.

The structure within the blood collecting apparatus 10 for swinging the above-described bag supporting plate 19 and the structure for measuring the weight of the blood bag 1 within the same are as follows.

A base 20 is provided on the bottom of the blood collecting evacuated chamber 13. The base 20 supports, through a supporting shaft 21, a swingable frame 22 capable of swinging. A swinging motor 23 is fixed to the base 20, and a drive shaft 24 driven by the swinging motor 23 is supported by the base 20. Reference numerals 25 and 26 denote toothed pulleys, while reference numeral 27 denotes a toothed belt. A crank wheel 28 is fixed to one end of the drive shaft 24. A link 29 is connected, at one end thereof, to a position on the radius of rotation of the crank wheel 28, the other end of the link 29 being connected to a link piece 30 integral with the swingable frame 22.

A pair of weigher mounting blocks 31 are fixed to the upper surface of the swingable frame 22, and a weigher (weight measuring means) 33 is cantilevered by a supporting plate 32 disposed across the end portions of these mounting blocks 31. The weigher 33 is provided with strain gauges 34 which are attached to two positions on the upper surface of the weigher and two positions on the lower surface of the same in such a manner as to form a Wheatstone bridge circuit, and which serve as a weight sensor. The bag supporting plate 19 is fixed to the tip portion of the weigher 33 through a weighing table 35 and a supporting plate 36. Reference numeral 37 denotes a stopper for preventing lateral vibration of the weigher 33, and reference numeral 38 denotes a weight sensor amplification unit.

With the above-described construction, within the blood collecting apparatus 10, the operation of the swinging motor 23 causes the rotation of the drive shaft 24 and the crank wheel 28, whereby the swingable frame 22 is swung, in turn causing the swinging of the bag supporting plate 19 supported by the swingable frame 22 through the weigher 33. Also within the blood collecting apparatus 10, the bag supporting plate 19 is supported by the weigher 33 cantilevered by the swingable frame 22 through the mounting blocks 31 and the supporting plate 32, whereby the weight of the blood bag 1 is measured on the basis of the change in the output of the strain gauges 34 in response to the flexural deformation of the weigher 33, so as to measure the amount of collected blood.

The blood collecting apparatus 10 also has the following arrangement. The rotational position of a detection cam 39 provided at the second end of the drive shaft 24 is detected by an optical sensor 40, so as to control the driving of the swinging motor 23. This allows the bag supporting plate 19 to be temporarily stopped at its lowermost descent point (the bottom dead center) and be held in its state of maintaining a certain attitude, in which condition, the weight of the blood bag 1 is measured in the above described manner.

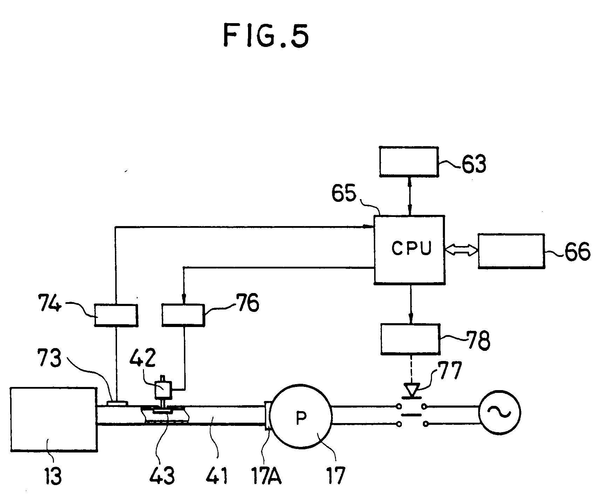

As shown in Fig. 5, within the blood collecting apparatus 10, the suction port 17A of the vacuum pump 17 is connected to the blood collecting evacuated chamber 13 via a vacuum pipe 41. A solenoid valve 43 is provided at an intermediate portion of the vacuum pipe 41, which valve closes when an evacuation solenoid 42 is energized, whereas it opens under weight when the evacuation solenoid 42 is deenergized. The blood collecting apparatus 10 performs the on/off control of the vacuum pump 17 so as to form a certain negative pressure (a certain degree of vacuum ) within the blood collecting evacuated chamber 13. At the completion of blood collection, the evacuation valve 43 is opened, thereby opening the blood collecting evacuated chamber 13 to the atmosphere.

The blood collecting apparatus 10 further includes a tube holder 44 on a front-side upper portion of the housing 11 which is adjacent to the blood collecting evacuated chamber 13, so as to enable the pulling out a blood collecting tube 2 communicating with the blood bag 1 accommodated in the blood collecting evacuated chamber 13. The tube holder 44 is provided with a tube clamp (blood collection stopping means) 46 driven by a tube clamp solenoid 45. The tube clamp 46 is operable to clamp and press the blood collecting tube 2 until the tube is closed so as to stop the action of collecting blood into the blood bag 1. Reference numeral 47 denotes a clamp release button for the tube clamp 46, and reference numeral 48 denotes a clamp button for actuating the tube clamp 46 in emergency.

The display panel 12 of the blood collecting apparatus 10 has a lamp 49 for switchingly displaying the collected blood amount/vacuum degree, a collected blood amount/vacuum degree selection switch 50, a lamp 51 for switchingly displaying the blood collection amount in ml (400 ml/200 ml), a 400 ml/200 ml selection switch 52, a stop switch 53, a start switch 54, a lamp 55 for displaying the bag in use, a bag-in-use selection switch 56, and a portion 57 for displaying the collected blood amount/vacuum degree. The blood collecting apparatus 10 further includes a power switch 58 and a fuse holder 59 which are at lower portions on the front surface of the housing 11, as well as a power source connector 60 at a lower portion on the reverse surface of the housing 11.

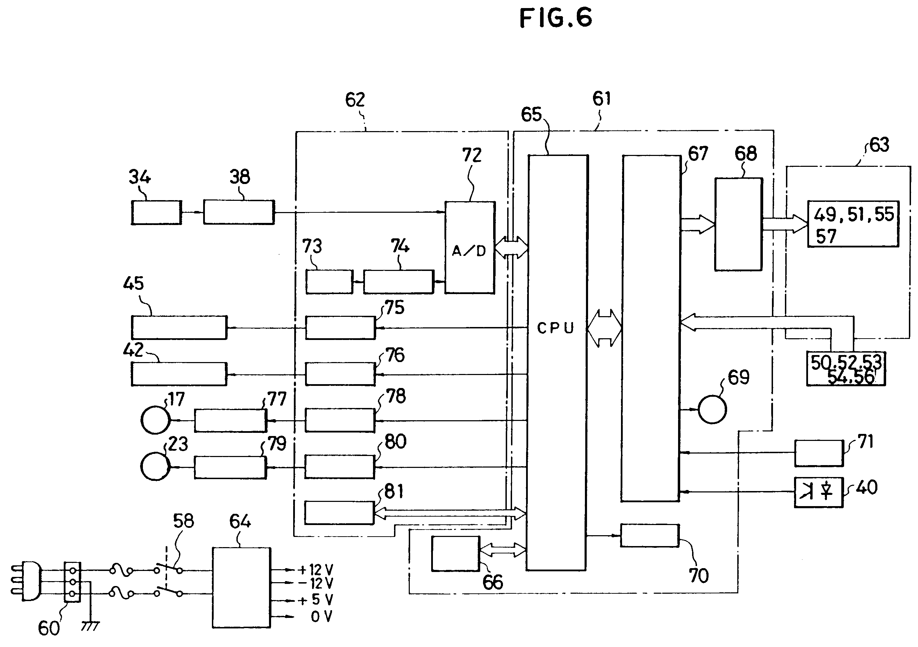

Next, a description of the control device 18 of the blood collecting apparatus 10 will be given. As shown in Fig. 6, the control device 18 mainly comprises a main control circuit 61, a drive Circuit 62, and a display Circuit 63. A power source unit is denoted by 64.

The main control circuit 61 has a CPU (central processing unit) [including a memory into which a control program for effecting a series of operations of the apparatus 10 is written] 65, a memory (storing means) 66, an input/output control section 67, a LED (light emitting diode) drive circuit 68, a buzzer 69, and a fail-safe circuit 70. Detection signals from the above-mentioned optical sensor 40 for detecting the swinging position of the bag supporting plate 19 and from a blood leakage sensor 71 for detecting leakage of blood from the blood bag 1 are transmitted to the input/output control section 67.

The above-mentioned memory 66 comprises a non-volatile memory such as an EA-ROM or EEP-ROM, and it allows the rewriting and the reading of stored data, while being capable of holding stored data without any application thereon of power source voltage. The memory 66 stores data such as 10 the negative pressure to be generated in the blood collecting evacuated Chamber 13, 02 the set amount of blood to be collected in the blood bag 1, ③ the reference time during which the vibration of the blood bag 1 is to be stopped at the blood collection terminating stage, and ④ specific constants [a, b, c and d] of sensors as will be described hereinafter.

The buzzer 69 generates sounds in one of different sound-generating manners in accordance with, e.g., ① the completion of blood collection, ② the occurrence of an error in the negative pressure generated in the blood collecting evacuated chamber 13, ③ the occurrence of an error in the rotation of the swinging motor 23, and ④ the detection of blood leakage by the blood leakage sensor 71.

The fail-safe circuit 70 checks the occurrence of runaway of the CPU 65, and, at the time of runaway, stops the apparatus in its safe condition.

The drive circuit 62 is connected to the main control circuit 61, and it has an A/D conversion circuit 72. The weight sensor amplification unit 38, to which the above-described strain gauges 34 are connected, is connected to the A/D conversion circuit 72. A pressure sensor 73 provided in the above-described vacuum pipe 41 in order to detect the negative pressure within the blood collecting evacuated chamber 13 is connected to the A/D Conversion circuit via a pressure sensor amplification circuit 74.

With this arrangement, the CPU 65 of the control device 18 calculates the weight of collected blood on the assumption that the output y of the strain gauges 34 is in a certain relationship with the measured weight w of collected blood, which can be expressed as a linear function y = aw + b. Also, on the assumption that the output z of the pressure sensor 73 is in a certain relationship with the measured pressure p, which can be expressed as a linear function z = cp + d, the CPU 65 calculates this pressure.

The drive circuit 62 also has ① a solenoid drive circuit 75 for controlling the tube clamp solenoid 45, ② a solenoid drive circuit 76 for controlling the evacuation solenoid 42, ③ a pump drive circuit 78 for turning on/off a power supply switch 77 for the vacuum pump 17, and ④ a motor drive circuit 80 for turning on/off a power supply switch 79 for the swinging motor 23.

The CPU 65 of the control device 18 receives the detection output of the pressure sensor 73 and the set pressure within the blood collecting evacuated chamber 13, which is among data stored in the memory 66, and it on/off controls the power supply swich 77 for the vacuum pump 17, as stated before, in such a manner that detected pressure coincides with the set pressure. This operation causes the negative pressure within the blood collecting evacuated chamber 13 to undergo fine changes within a fixed range of the set pressure, so that, as a result, a constant pressure condition is achieved.

Next, a description will be given of the procedure for the blood collecting operation performed by the blood collecting apparatus 10.
① The power switch 58 is turned on.
② The amount of blood to be collected is selected by means of the 400 ml/200 ml selection switch 52. The result of this selection is displayed by the switchingly displaying lamp 51.
③ The bag to be used is selected by means of the bag-in-use selection switch 56. The result of this selection is displayed by the display lamp 55. The bag in use may be one of the following types: the single (S) type solely comprising the main bag, and types further comprising at least one small bag, i.e., the double (D) ,type, the triple (T) type, and the quadruple (Q) type.
④ A blood collecting needle provided at the tip of the blood collecting tube 2 is stuck into the donor, and this is followed by the collection of blood to a certain extent.
⑤ The blood collecting bag 1 is received in the blood collecting evacuated chamber 13 and is placed on the bag supporting plate 19. The blood collecting tube 2 is set in the tube holder 44. The cover 14 is shut.
⑥ The start switch 54 is turned on. The control device 18 controls the driving of the vacuum pump 17 and the swinging motor 23 in such a manner that blood is collected with pressure reduction in the blood collecting evacuated chamber 13, while the bag supporting plate 19 is swung. The control device 18 also operates, at a timing at which the bag supporting plate 19 temporarily stops at its lowermost descent point, to receive the output of the weight sensor amplification unit 38, to detect the measured amount of blood collected in the blood bag 1, and to calculate the amount (volume) of blood yet to be collected, by using the set blood collection amount, the specific gravity of blood, and the previously-registered weight of the blood bag 1, these data being written in the memory 66, and using the following equation (1 ):
   Yet-to-be-collected blood amount (ml) = [set blood collection amount (g) + previously-registered weight (g) - collected blood measured amount (g)]/specific gravity (g/ml) (1)
⑦ The control device 18 causes the tube clamp 46 to clamp and press the blood collecting tube 2 until the tube is closed so as to stop the action of collecting blood in the blood bag 1 under the condition that the remaining amount of collected blood obtained as the result of the abovementioned calculation has reached zero. At this time, the control device 18 is operated to stop the vacuum pump 17 and to open the exhaust valve 43 for opening the blood collecting evacuated chamber 13 to the atmosphere.
⑧ After the completion of the above-described blood collection, the control device 18 causes a re-driving of the swinging motor 23 for a certain time, so as to again swing the bag supporting plate 19. Thereafter, the buzzer posts the completion of blood collection.
@ The clamp release button 47 is turned on, the cover 14 is opened, the blood collecting tube 2 is removed from the tube holder 44, and the blood collecting bag 1 is taken out of the blood collecting evacuated chamber 13.

In the following a first embodiment is described.

In the CPU 65 of the control device 18, the characteristics of the strain gauges 34 and the characteristic of a pressure sensor 73 are corrected in an early stage and in a transit stage of the use of the blood collecting apparatus 10 through procedures (A) and (B) set forth hereunder.
(A) The output yi of the strain gauges 34 in two pieces of the respective known weight wi of collected blood is taken up and the constants a and b constituting the above-mentioned function y = aw + b are corrected on the basis of such data taken up. Concretely, this correction is made in accordance with the following proce- _{d}ures (D - .
   10 The acting mode of the CPU 65 is set to the mode for detecting and correcting the weight by a mode selection switch 81 which the drive circuit 62 is provided with, in the state where the power switch 58 is turned on and the blood collection has not yet started or the blood collection has already been completed (the weight of collected blood is zero).
   ② The CPU 65 stores the output y1 (w = 0) of the strain gauges 34 when w = 0 as described in ①.
   ③ The operator puts a known weight of, for instance, w=500g on the bag supporting plate 19. At this time, the operator sets the display value of the display portion 57 to "500g" (the display value is reduced by actuation of the stop switch 53 and increased by actuation of the start switch 54), so that the, output y2 (w = 500) of the strain gauges 34 is made enter the CPU 65 by actuation of the selection switch 50 when w = 500g. @ The CPU 65 calculates the constants a and b of the linear function y=aw + b from two data y1 (w = 0) and y2 (w = 500) of the above ② and ③ and writes the results of the calculation into the memory 66. At this time, the buzzer 69 generates sounds.
   ⑤ Thereafter, the operator puts another known weight of, for example, w=800g on the bag supporting plate 19. The CPU 65 calculates the weight w of this time using a and b calculated in the above @ and the output y3 of the stain gauges 34 of this time and displays the result w3 of this calculation on the display portion 57. If the display value of the display portion 57 becomes "800g", the correction is completed, and if the display value is different from "800g", the CPU 65 judges that the sensor itself or the surroundings of the sensor are something wrong and corrects the same.
(B) The output zi of the strain gauges 34 in two pieces of the respective known pressure pi is taken up and the constants c and d constituting the above-mentioned function z = cp + d are corrected on the basis of such data taken up. Concretely, this correction is made in accordance with the following procedures ①~⑤ .
   10 The acting mode of the CPU 65 is set to the mode for detecting and correcting the pressure by a mode selection switch 81 which the drive circuit 62 is provided with, in the state where the power switch 58 is turned on and the blood collection has not yet started or the blood collection has already completed (the negative pressure in the blood collecting evacuated chamber 13 is zero).
   ② The CPU 65 stores the output z1 = 1 (p = 0) when p = 0 in the above 10.
   ③ Then, the CPU 65 automatically actuates the vacuum pump 17 to form a saturated evacuated pressure state in the collecting blood evacuated chamber 13. The operator considers the negative pressure, for example, p=-150mmHg, in the blood collecting evacuated chamber 13 as already known by a manometer inserted through the blood collecting evacuated chamber 13. The operator sets the display value of the display portion 57 to "-150mmHg" (the display value is reduced by the actuation of the stop switch 53 and increased by the actuation of the start switch 54), so that the output z2 (p=-150) of the pressure sensor 73 is made enter the CPU 65 by actuation of the selection switch 50 when p=-150mmHg.
   @ The CPU 65 calculates the constants c and d of the linear function z=cp + d from two data z1 (p = 0) and z2 (p = -150) of the above ② and ③ and writes the results of the calculation into the memory 66. At this time, the buzzer 69 generates sounds.
   ⑤ Thereafter, the operator forms a new saturated evacuated pressure in the blood collecting evacuated chamber 13 by releasing pressure from an intermediate portion of the vacuum pipe 41 or by other means and obtains, for example, p=-100mmHg through the manometer as in the above ③. The CPU 65 calculates the pressure p of this time using c and d calculated in the above @ and the output z3 of the stain gauges 34 of this time and displays the result p3 of this calculation on the -display portion 57. If the display value of the display portion 57 becomes "-100mmHg", the correction is completed, and if the display value is different from "-100mmHg", the CPU 65 judges that the sensor itself or the surroundings of the sensor are wrong and corrects the same.

Next, the operation of the above-mentioned first embodiment will be described.

The constants a and b of the function y = aw + b expressing that the output y of the strain gauges 34 is in a certain relationship with the measured weight w of collected blood can be corrected through the above procedure (A). That is, the characteristic of the sensor for measuring the amount of collected blood can be corrected with ease and the accuracy in measurement of the amount of collected blood can be obtained without requiring the conversion circuit and volume.

The constants c and d of the function z = cp + d expressing that the output z of the pressure sensor 73 is in a certain relationship with the negative pressure p of the blood collecting evacuated chamber 13 can also be corrected through the above procedure (B). That is, the characteristic of the pressure sensor can also be corrected with ease and the accuracy in measurement of pressure generated within the blood collecting evacuated chamber can also be obtained without requiring the conversion circuit and volume.

In the above-mentioned first embodiment, in ③of the above procedure (A) and in ③ of the above procedure (B), when the operator makes the known weight wi of collected blood and the known pressure pi enter the CPU 65, the entered values are displayed on the display portion 57. Accordingly, the operation of the operator and the values entered into the CPU 65 can visually be recognized. In the following a second embodiment is described.

In the CPU 65 of the control device 18, the offset value b of the strain gauges 34 and the offset value d of the pressure sensor 73 are corrected in the state where the power switch 58 is turned on and the blood collection has not yet started or the blood collection has already been completed (the weight of collected blood is zero and the negative pressure in the blood collecting evacuated chamber 13 is also zero) through the procedures (A) and (B) set forth hereunder.
(A) The CPU 65, as mentioned above, automatically takes up the output y0 of the strain gauges 34 every predetermined cycle in the state where the measured weight w of collected blood is zero and corrects b of the above-mentioned function y = aw + b on the basis of such y0 taken up. Concretely, with reference to the off- set value b01 which was already written into the memory 66 in the preceding correction stage, if the above-mentioned y0, which is in the correction stage of this time, satisfies the expression 10 y0 b01 + m (for example, m = 3g), the CPU 65 adopts the above-mentioned y0 as the new offset value and stores the same in the memory thereof, and if it satisfies the expression 0 y0 > b01 + m, the CPU 65 judges that a substance is loaded on the sensor and adopts b01 of the preceding time also for this time.
(B) The CPU 65, as mentioned above, automatically takes up the output z0 of the pressure sensor 73 every predetermined cycle in the state where the negative pressure p in the blood collecting evacuated chamber 13 is zero and corrects the above-mentioned function z = cp + d on the basis of such z0 taken up. Concretely, with reference to the off-set value d01 which was already written into the memory 66 in the preceding correction stage, if the above- mentioned z0, which is in the correction stage of this time, satisfies the expression 10 z0 d01 + r (for example, r=3mmHg), the CPU 65 adopts the above-mentioned z0 as the new off- set value and stores the same in the memory thereof, and if it satisfies the expression 0 z0 > z01 + r, the CPU 65 judges that pressure changes have already taken place owing to the start of blood collection and maintains d01 of the preceding time also for this time.

Next, the operation of the above-mentioned second embodiment will be described.

The offset value b of the function y =aw + b expressing that the output y of the stain gauges 34 is in a certain relationship with the measured weight w of collected blood can automatically be corrected every predetermined cycle through the the above-mentioned procedure (A). Accordingly, the change of the offset value b in the output of the stain gauges 34 caused by drift can normally and automatically be corrected and the accuracy in measurement of the weight of collected blood can be obtained.

Similarly, the offset value d of the function z = cp + d expressing that the output z of the pressure sensor 73 is in a certain relationship with the measured Pressure p is also automatically corrected every predetermined cycle through the above-mentioned procedure (B). Accordingly, the changes of the offset value d of the output of the pressure sensor 73 caused by drift can also normally and automatically be corrected and the accuracy in measurement of the pressure to be generated in the blood collecting evacuated chamber 13 can also be obtained.

According to the present invention, the characteristic of the sensor for measuring the amount of collected blood can be corrected with ease and the accuracy in measurement of the amount of collected blood can be obtained.

According to the present invention, the characteristic of the pressure sensor can also be corrected with ease and the accuracy in measurement of the pressure to be generated in the blood collecting evacuated chamber can also be obtained.

According to the present invention, the changes of the offset value of the output of the sensor for measuring the amount of collected blood caused by drift can normally and automatically be corrected, and the accuracy in measurement of the amount of collected blood can be obtained.

The present invention is basically likewise applicable even if the above-mentioned functions y = f(w) and z = f(p) are not linear functions but quadric functions, index functions and logarithms.

## Claims

1. A blood collecting apparatus (10), wherein the blood container (1) is set in blood collecting evacuated chamber (13) and blood is collected into the blood container by negative pressure generated in the blood collecting evacuated chamber, the apparatus further being provided with a correcting function of a sensor (33) including weight measuring means provided with a sensor for measuring the amount of collected blood when blood is collected in the blood container (1), and control means (65) for calculating the amount of collected blood on the assumption that the output y of the measuring sensor is in a certain relationship with the measured weight w of collected blood, which can be expressed as a function y = f-(w) comprising at least one constant value (a, b),
characterised in that
- the control means (65) is provided with a memory (66) for storing specific constant values (a, b) of the sensor (33),
- the control means (65) is provided with a correction mode, which may be adopted before starting or after completion of blood collection, in which correction mode n + 1 pieces of known amounts wi of collected blood are measured by the sensor (33) and the output yi of the sensor is taken by the control means for correcting the constant value (a, b) of the function y = f(w) and the corrected constant value is rewritten in the memory (66), thus correcting the characteristic of the sensor (33),
- the control means (65) is provided for calculating the weight of the collected blood using the corrected constant values (a, b),
- a pressure measuring means is provided with a pressure sensor (73) for detecting the pressure in the blood collecting evacuated chamber (13), the control means (65) calculating pressure on the assumption that the output z of the pressure sensor is in a certain relationship with the measured pressure p, which can be expressed as a function z = f(p), comprising at least one constant value (c, d), the memory (66) of the control means (65) storing the corresponding specific constants (c, d) of the pressure sensor (73), and
- in the correction mode of the control means n + 1 pieces of known pressure amounts pi are measured by the pressure sensor (73) and the output zi is taken by the control means (65) for correcting the constant value (c, d) and the corrected constant value is rewritten in the memory (66), thus correcting the characteristic of the pressure sensor.

2. The blood collecting apparatus (10) of claim 1, provided with an automatic correction function of the sensor (33), characterised in that the output y0 of the measuring sensor is automatically taken up every predetermined cycle in the state where the measured weight w of collected blood is zero and the off-set value of the function y = f(w) is corrected on the basis of y0.

3. The blood collecting apparatus (10) of claim 1 or 2, wherein the measuring sensor is a weight sensor (33) for measuring the weight of collected blood.

## Patentansprüche

1. Blutsammelvorrichtung (10), worin der Blutbehälter (1) in eine evakuierte Blutsammelkammer (13) gebracht wird, und Blut in den Blutbehälter durch Unterdruck, der in der evakuierten Blutsammelkammer erzeugt wird, gesammelt wird, wobei die Vorrichtung weiter versehen ist mit einer Korrekturfunktion eines Sensors (33) , der eine Gewichtsmeßeinrichtung mit einem Sensor zum Messen der Menge des gesammelten Blutes einschließt, wenn Blut im Blutbehälter (1) gesammelt wird, und mit einer Steuereinrichtung (65) zum Berechnen der Menge des gesammelten Blutes unter der Annahme, daß das Ausgabesignal y des Meßsensors in einer bestimmten Beziehung zum gemessenen Gewicht w des gesammelten Blutes steht, die durch eine Funktion y = f(w) ausgedrückt werden kann und die mindestens einen konstanten Wert (a, b) umfaßt,
gekennzeichnet dadurch, daß
. eine Steuereinrichtung (65) mit einem Speicher (66) zum Speichern spezifischer, konstanter Werte (a, b) des Sensors (33) bereitgestellt wird,
. die Steuereinrichtung (65) mit einem Korrekturmodus versehen ist, der vor dem Starten oder nach Beendigung der Blutsammlung angenommen werden kann, wobei in diesem Korrekturmodus n + 1 Portionen bekannter Mengen w, von gesammeltem Blut durch den Sensor (33) gemessen werden, und das Ausgabesignal y des Sensors durch die Steuereinrichtung zum Korrigieren des konstanten Wertes (a, b) der Funktion y = f(w) verwendet wird, und der korrigierte, konstante Wert in den Speicher (66) zurückgeschrieben wird, wodurch die Charakteristik des Sensors (33) korrigiert wird,
. die Steuereinrichtung bereitgestellt wird zum Berechnen des Gewichtes des gesammelten Blutes unter Verwenden der korrigierten, konstanten Werte (a, b),
. eine Druckmeßeinrichtung mit einem Drucksensor (73) zum Feststellen des Druckes in der evakuierten Blutsammelkammer (13) bereitgestellt ist, wobei die Steuereinrichtung (65) den Druck unter der Annahme berechnet, daß das Ausgabesignal z des Drucksensors in einer bestimmten Beziehung zum gemessenen Druck p steht, die durch eine Funktion z = f(p) ausgedrückt werden kann, die mindestens einen konstanten Wert (c, d) umfaßt, wobei der Speicher (66) der Steuereinrichtung (65) die entsprechenden spezifischen, Konstanten (c, d) des Drucksensors (73) speichert, und
im Korrekturmodus der Steuereinrichtung n + 1 Messungen von bekannten Druckhöhen p durch den Drucksensor (73) durchgeführt werden, und das Ausgabesignal z durch die Steuereinrichtung (65) zum Korrigieren des konstanten Wertes (c, d) verwendet und der korrigierte Wert in den Speicher (66) zurückgeschrieben wird, wodurch die Charakteristik des Drucksensors korrigiert wird.

2. Blutsammelvorrichtung (10) nach Anspruch 1, die mit einer automatischen Korrekturfunktion des Sensors (33) versehen ist, dadurch gekennzeichnet, daß das Ansgabesignal yo des Meßsensors automatisch bei jedem festgelegten Zyklus in dem Zustand aufgenommen wird, in dem das gemessene Gewicht w des gesammelten Blutes gleich Null ist und der Offsetwert der Funktion y = f(w) auf Basis von yo korrigiert ist.

3. Blutsammelvorrichtung (10) nach Anspruch 1 oder 2, worin der Meßsensor einen Gewichtssensor (33) zum Messen des Gewichtes des gesammelten Blutes darstellt.

## Revendications

1. Appareil (10) de prélèvement de sang dans lequel le récipient à sang (1) est placé dans une chambre (13) de prélèvement de sang mise sous vide et du sang est recueilli dans le récipient à sang grâce à une pression négative produite dans la chambre de prélèvement de sang mise sous vide, l'appareil étant en outre muni d'une fonction de correction d'un capteur (33) comprenant un moyen de mesure de poids pourvu d'un capteur qui sert à mesurer la quantité de sang prélevé quand du sang est recueilli dans le récipient à sang (1) et des moyens de commande (65) pour calculer la quantité de sang prélevé en se basant sur la supposition que la sortie y du capteur de mesure est reliée au poids mesuré w de sang prélevé par une certaine relation qui peut être exprimée comme une fonction y = f(w) comprenant au moins une valeur constante (a, b),
caractérisé en ce que:
- le moyen de commande (65) est pourvu d'une mémoire (66) pour mémoriser des valeurs spécifiques des constantes (a, b) du capteur (33),
- le moyen de commande (65) est pourvu d'un mode correction qui peut être adopté avant le début du prélèvement de sang ou une fois qu'il est terminé, mode de correction dans lequel n + 1 pièces de quantités connues wi de sang prélevé sont mesurées par le capteur (33) et la sortie yi du capteur est utilisée par le moyen de commande pour corriger la valeur des constantes (a, b) de la fonction y=f(w) et la valeur des constantes corrigées est réécrite dans la mémoire (66), ce qui corrige la caractéristique du capteur (33),
- le moyen de commande (65) sert à calculer le poids du sang prélevé à l'aide des valeurs corrigées des constantes (a, b),
- un moyen de mesure de pression est muni d'un capteur de pression (73) destiné à détecter la pression dans la chambre (13) de prélèvement de sang mise sous vide, le moyen de commande (65) calculant la pression en se basant sur la supposition que la sortie z du capteur de pression est reliée à la pression mesurée p par une certaine relation qui peut être exprimée comme une fonction z=f(p) comprenant au moins une valeur constante (c, d), la mémoire (66) du moyen de commande (65) mémorisant les constantes spécifiques correspondantes (c, d) du capteur de pression (73), et
- lorsque le moyen de commande est en mode correction, n + 1 pièces de quantités de pression connues pi sont mesurées par le capteur de pression (73) et la sortie zi est utilisée par le moyen de commande (65) pour corriger la valeur des constantes (c, d) et la valeur des constantes corrigées est réécrite dans la mémoire (66), ce qui corrige la caractéristique du capteur de pression.

2. Appareil (10) de prélèvement de sang selon la revendication 1, pourvu d'une fonction automatique de correction du capteur (33), caractérisé en ce que la sortie y0 du capteur de mesure est automatiquement relevée pour chaque cycle prédéterminé lorsque le poids mesuré w du sang prélevé est nul et la valeur à l'origine de la fonction y = f(w) est corrigée sur la base de y0.

3. Appareil (10) de prélèvement de sang selon la revendication 1 ou 2, dans lequel le capteur de mesure est un capteur de poids (33) destiné à mesurer le poids du sang prélevé.
